# EUROPEAN PATENT APPLICATION

(11) **EP 0 603 976 A2**
(43) Date of publication of application: **29.06.1994**
(21) Application number: 93203631.2
(22) Date of filing: 21.12.1993
(51) Int. Cl.: A61F 2/30, A61L 27/00, B29C 45/14, A61F 2/36

(54) **Partially resorbable implant**

(30) Priority: 21.12.1992 US 993538
(71) Applicant: Bristol-Myers Squibb Company, New York, N.Y. 10154 (US)
(72) Inventor: Lin, Steve, Ft. Wayne, IN 46804 (US)
(74) Representative: Mays, Julie

(57) **Abstract**

A partially resorbable implant (10) having a sleeve (19) of porous material (18) about the resorbable material to promote bone growth within the material and thereby enhance long term fixation of the implant (10). In the preferred embodiment, the porous material (18) is a fiber metal pad which has a long history of providing an exemplary structure for bone to grow into. When implanted, as the resorbable stem portion (14) of the implant (10) resorbs, bone may grow into the porous sleeve (19). Therefore, the resulting implant (10) will have fixation at the point of the non-resorbable proximal portion (12) of the implant (10) as well as along the length of the porous sleeve (19).

## Description

### Field of the Invention

This invention relates to a prosthetic implant having a portion which is resorbable within the body surrounded by a fiber metal layer which is not resorbable within the body but which provides long term fixation.

### Background of the Invention

Prosthetic implants are well known for the replacement of at least a portion of a human joint. Such implants, in the past, have been formed generally from metals which are compatible within the human body. Fully metal implants may stress shield the supporting bone causing its resorption. However, recently, it has become known to utilize a resorbable material for at least a stem portion of the implant thus minimizing stress shielding from the stem. In U.S. patent 4,990,161 issued to Kampner on February 5, 1991, the problems associated with fully metal implants and the advantages of a partially resorbable implant is fully discussed. The Kampner patent 4,990,161 is hereby incorporated by reference.

A partially resorbable implant as illustrated in the Kampner patent may exhibit fixation problems after the resorbable portion has resorbed leaving only the non-resorbable portion in contact with the bone.

### Summary of the Invention

The partially resorbable implant of this invention eliminates the problems discussed above by providing a sleeve of porous material about the resorbable material to promote bone growth within the material and thereby enhance long term fixation of the implant. In the preferred embodiment, the porous material is a fiber metal pad which has a long history of providing an exemplary structure for bone to grow into. When implanted, as the resorbable portion of the implant resorbs, bone may grow into the porous sleeve. Therefore, the resulting implant will have fixation at the point of the non-resorbable portion of the implant as well as along the length of the porous sleeve. Since sleeve of porous metal remaining after resorption is quite flexible, stress shielding due to the stem is eliminated.

Accordingly, it is an advantage of the invention to provide an implant having a portion which is resorbable within the body which is surrounded by a layer of porous material suitable for bone ingrowth.

Another object of the invention is to provide a prosthetic implant having a layer of fiber metal mesh carried by a resorbable portion which provides fixation of the implant after the resorbable portion has resorbed.

Other advantages of the invention will become apparent upon a reading of the following description taken with the accompanying drawing.

### Brief Description of the Drawing

Fig. 1 is an elevational view with portions cut away for illustrative purposes of the implant of the invention.

Fig. 2 is an enlarged sectional view of the area circled in Fig. 1 and designated by the reference numeral 2.

### Description of the preferred Embodiment

The preferred embodiment herein described is not intended to be exhaustive or to limit the invention to the precise form disclosed. Rather, it is chosen and described to best explain the invention so that others skilled in the art might utilize its teachings.

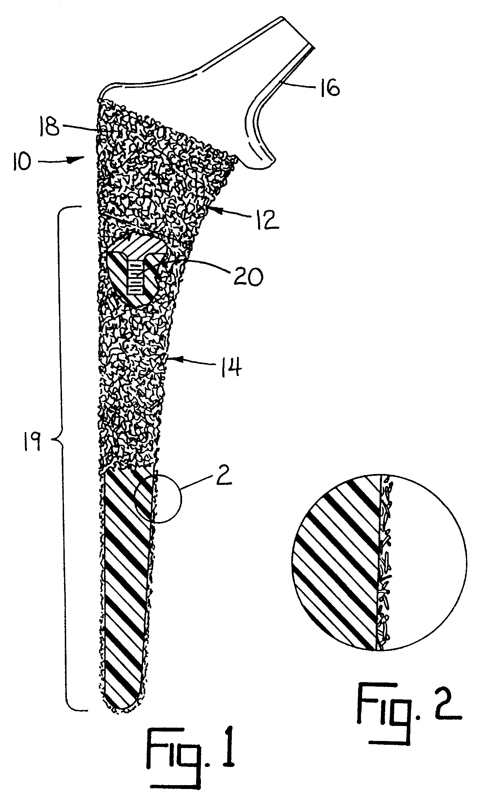

Referring to Fig. 1, a prosthetic hip stem 10 constructed in accordance with this invention is illustrated. Hip stem 10 includes a proximal body 12 and a distal stem 14. A neck 16 extends from proximal body 12 and is configured to accommodate a prosthetic femoral head, not shown. The proximal body 12 and stem 14 are covered substantially by a layer 18 of porous metal preferably in the form of a fiber metal mesh pad (see Fig. 3). On the distal stem 14 the layer 18 of porous metal forms a sleeve 19. Stem 14 is made from a material which is resorbable within the body after implantation but which exhibits sufficient strength characteristics to be implantable and provide initial fixation. A more thorough explanation of resorbable materials and the advantages over a completely metal implant may be had by reference to the incorporated Kampner patent. For the purpose of this invention any material which in its solid form is rigid and yet may be absorbed by the body without harm thereto is applicable.

The proximal body 12 of implant 10 is formed from a solid metal and includes a screw shank 20 extending as illustrated in the Figures. Shank 20 provides a locking surface to connect the resorbable stem portion to the proximal body. In practice, the proximal body with the porous layer attached is placed within an injection mold machine. The resorbable material is injected into the mold and interdigitates within the porous metal and shank to form the stem and firmly connect the stem to the proximal body.

In use, after the stem is implanted, the fluids within the body cause the stem to resorb exposing the porous metal layer to the surrounding bone. As the stem resorbs, the bone begins to interdigitate or grow into the fiber layer around the stem and proximal body. After full resorption of the stem 14, only the sleeve of porous metal and the proximal body remains in the canal of the bone. Since the porous metal is naturally flexible, stress shielding of the bone is eliminated. Further with bone growing into the fiber metal sleeve fixation of the implant is increased.

It should be understood that while the subject invention is described in relation to a prosthetic hip stem implant, such should not be considered a limitation on the invention. The invention is applicable to any orthopaedic implant using a bioresorbable material.

Further it should be understood that the invention is not to be limited to the precise description above but may be modified within the scope of the appended claims.

## Claims

1. An implant comprising an elongated member for insertion into a bone cavity for fixing the implant relative to the bone, the member being constructed of a resorbable material carrying a porous layer for supporting interdigitation of bone growth.

2. The implant of Claim 1 wherein said implant further includes a non-resorbable body portion connected to the elongated member.

3. A prosthetic hip stem implant having a proximal body and a stem extending away from said body, said proximal body being formed from a non-resorbable material, said stem being formed from a biodegradable material which can resorb after a period of time, a layer of porous material being carried by said stem and body, said porous material providing a structure for bone growth therein after said biodegradable material has at least partially resorbed.

4. A method of making an orthopaedic implant having an elongated stem portion, the method comprising the steps of:
a) providing a solid non-resorbable body portion;
b) providing a layer of porous material;
c) wrapping said layer of porous material around said body portion such that a portion of said porous material forms a sleeve;
d) filling said sleeve with a biodegradable material which resorbs after a period of time.
